# EUROPÄISCHE PATENTSCHRIFT

(11) **EP 1 474 119 B1**
(45) Veröffentlichungstag und Bekanntmachung des Hinweises auf die Patenterteilung: **04.06.2008**
(21) Anmeldenummer: 03706473.0
(22) Anmeldetag: 10.02.2003
(51) Int. Cl.: A61K 31/00, A61K 36/53, A61P 3/04, A61P 5/30, A61P 5/34, A61P 15/12

(54) **VERWENDUNG VON SELEKTIV DEN ESTROGENREZEPTOR BETA MODULIERENDEN PHYTOESTROGENHALTIGEN EXTRAKTEN**
USE OF EXTRACTS CONTAINING PHYTOESTROGEN SELECTIVELY MODULATING ESTROGEN-RECEPTOR-BETA
UTILISATION D'EXTRAITS CONTENANT DES PHYTOOESTROGENES MODULANT DE MANIERE SELECTIVE LE RECEPTEUR BETA D'OESTROGENES

(30) Priorität: 15.02.2002 DE 10206390
(43) Veröffentlichungstag der Anmeldung: 10.11.2004
(73) Patentinhaber: Bionorica AG, 92318 Neumarkt (DE)
(72) Erfinder: WUTTKE, Wolfgang, 37120 Bovenden (DE); JARRY, Hubertus, 37249 Neu-Eichenberg (DE); CHRISTOFFEL, Volker, 92369 Buchberg (DE); SPENGLER, Barbara, 92318 Neumarkt (DE); POPP, Michael, 91207 Lauf (DE)
(74) Vertreter: Winter, Brandl, Fürniss, Hübner Röss, Kaiser, Polte Partnerschaft Patent- und Rechtsanwaltskanzlei
(86) Internationale Anmeldenummer: PCT/EP2003/001311
(87) Internationale Veröffentlichungsnummer: WO 2003/068199

(56) Entgegenhaltungen:
- WO-A-01/74345
- WO-A-02/074321
- US-A- 5 952 374
- US-B1- 6 326 366
- C. GORKOW: "Klinischer Kenntnisstand von Agni-casti fructus" ZEITSCHRIFT FÜR PHYTOTHERAPIE, Bd. 20, - 1999 Seiten 159-168, XP009009970
- LIU J ET AL: "EVALUATION OF ESTROGENIC ACTIVITY OF PLANT EXTRACTS FOR THE POTENTIAL TREATMENT OF MENOPAUSAL SYMPTOMS" JOURNAL OF AGRICULTURAL AND FOOD CHEMISTRY, AMERICAN CHEMICAL SOCIETY. WASHINGTON, US, Bd. 49, Nr. 5, 2001, Seiten 2472-2479, XP001068359 ISSN: 0021-8561
- MILEWICZ A ET AL: "VITEX AGNUS CASTUS-EXTRAKT ZUR BEHANDLUNG VON REGELTEMPOMALIEN INFOLGE LATENTER HYPERPROLAKINAEMIE ERGEBNISSE EINER RANDOMISIERTENPLAZEBO-KONTROLLIERTEN DOPPELBLINDSTUDIE" ARZNEIMITTEL FORSCHUNG. DRUG RESEARCH, EDITIO CANTOR. AULENDORF, DE, Bd. 43, Nr. 7, 1993, Seiten 752-756, XP000913700 ISSN: 0004-4172
- HOBERG E ET AL: "Diterpenoids from the fruits of Vitex agnus-castus" PHYTOCHEMISTRY, PERGAMON PRESS, GB, Bd. 52, Nr. 8, Dezember 1999 (1999-12), Seiten 1555-1558, XP004291171 ISSN: 0031-9422

## Beschreibung

Die vorliegende Erfindung betrifft die Verwendung von phytoestrogenhaltigen Extrakten, die selektiv den Estrogen-Rezeptor-beta [ER-beta] modulieren ohne eine uterotrope Wirkung aufzuweisen gemäß dem Oberbegriff des Anspruchs 1.

Extrakte aus Vitex Agnus Castus (Mönchspfeffer, Keuschlamm) sind seit längerer Zeit zur Behandlung von Regeltempoanomalien, Mastodynie, prämenstruellem Syndrom, Störungen der Regelblutung (infolge primärer u. sekundärer Gelbkörperinsuffizienz) im Gebrauch, jedoch wurden derartige Extrakte nicht zur Estrogenersatztherapie eingesetzt.

In der Menopause kommt es zu einem Absinken der Estradiolspiegel in Folge des Erlöschens der Ovarialfunktion. Dies resultiert in einer Abschwächung proliferativer Prozesse und führt im Hypothalamus zu einer Verstärkung der Aktivität des GnRH-Pulsgenerators. (Der Gonadotropin-Releasing-Hormon-Pulsgenerator ist eine Art Taktgeber im Hypothalamus und taktet die pulsatile Ausschüttung von LH, wobei Steroide die Amplitude und die Frequenz beeinflussen.) Die resultierende stimulierte LH-Ausschüttung führt bei der klimakterischen Frau zu störend empfundenen aufsteigenden Hitzwallungen, den sogenannten "Hot flushes".

In Abwesenheit genügend hoher Estradiolspiegel im Blut überwiegt im Knochengewebe die Aktivität der Osteoklasten und damit der Abbau der Knochenmasse, der mit erhöhter Bruchgefahr des Skelettes einhergeht. Gleichzeitig besteht langfristig die Gefahr der Plaquebildung im Gefäßsystem und damit das erhöhte Risiko von Infarkten.

Nachteilig wären estrogenartige Wirkungen auf Uterus, Vagina, Brustgewebe und Leber. Unerwünscht hieran ist jedoch, dass bislang kein Phytopharmakon im Stand der Technik zur Verfügung stand, welches zu einer organselektiven Prophylaxe oder Therapie bei Estrogenmangel verwendet werden kann.

Dokument WO 01/74345 A2 beschreibt eine Reihe von Leguminosen als Phytoestrogenquelle zur Behandlung von Fettleibigkeit.

Ausgehend von diesem Stand der Technik ist es daher Aufgabe der vorliegenden Erfindung, pflanzliche Arzneimittel mit estrogenartiger Wirkung zur Verfügung zu stellen.

Die Lösung dieser Aufgabe erfolgt durch eine Verwendung gemäß Anspruch 1.

Mit den phytoestrogenhaltigen Extrakten, insbesondere aus Vitex Agnus castus, vorzugsweise Früchten, der vorliegenden Erfindung ist es erstmals möglich, eine selektive ER-beta-Wirkung - ohne uterotrope Wirkung - zu erzielen. Durch die verwendeten Vitex Agnus castus Extrakte ist es durch Modulation des ER-beta möglich, die folgenden Krankheitsbilder und pathophysiologischen Zustände zu behandeln:
- Fettleibigkeit und dadurch mögliche Beeinflussung des metabolischen Syndroms, insbesondere Hypertonus, Arteriosklerose, Herzinfarkt, Hyperandrogenämie

Die Extrakte zur erfindungsgemäßen Verwendung können in pharmazeutisch üblicher Form verwendet werden, beispielsweise in Form von Tropfen, Tabletten, Filmtabletten, Kapseln, Granulaten, Dragees, Suppositorien, Salben Cremes oder dgl..

Extrakte aus Vitex Agnus Castus, insbesondere deren Früchte und/oder Blätter sind erfindungsgemäß, erhältlich durch ethanolische Extraktion der Pflanze oder den Pflanzenteilen.

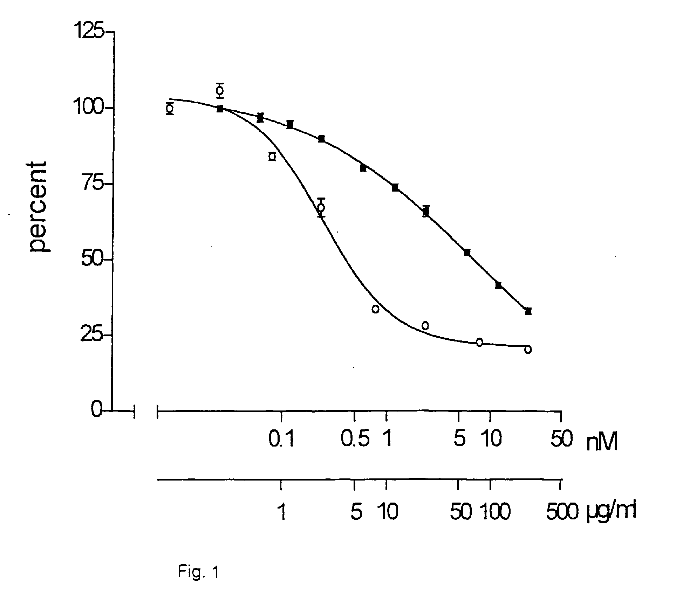

Um das Vorliegen von Phytoestrogenen in Vitex Agnus castus (VAC) - Extrakten zu zeigen, haben die Erfinder einen kompetitiven Estrogenrezeptor-Assay verwendet, bei welchem radioaktiv markiertes Estradiol von den Rezeptoren durch Liganden, die ebenfalls an den Rezeptor binden, im folgenden Rezeptormodulatoren genannt; verdrängt wird. Als Rezeptormodulatoren wurden einerseits nichtradioaktives Estradiol und andererseits der zu untersuchende Extrakt eingesetzt. Die Rezeptorpräparation bestand aus der cytosolischen Fraktion aus Schweineuteri, welcher beide Estrogenrezeptor-Subtypen, nämlich ER-alpha und ER-beta enthält. Die Ergebnisse eines typischen Experiments sind in Fig. 1 gezeigt.

Figur 1 zeigt die Verdrängungskurven von Estradiol (E₂, offene Symbole) und von VAC-Extrakten (dunkle Symbole). Die Ordinate gibt den prozentualen Anteil der (noch verbleibenden) Bindung von radioaktiv markiertem Estradiol an den Estrogenrezeptor an. Die obere Abszisse bezeichnet die E₂-Konzentration, während die untere Abszisse die VAC-Konzentration wiedergibt. Diejenige Verdrängung, welche mit der niedrigsten Konzentration der Testsubstanzen erhalten wurde, wird als 100% definiert. Mittelwerte ± SEM wurden aus einer Dreifachbestimmung erhalten. Die EC₅₀ von VAC beträgt 7.8 µg/ml

Wie aus Fig. 1 ersichtlich, konkurrieren die Verbindungen in den VAC-Extrakten dosisabhängig mit radioaktiv markiertem Estradiol um die Bindungsstellen der Estrogenrezeptoren . Die erhaltenen Daten demonstrieren eindrucksvoll, daß die untersuchten Extrakte Phytoestrogene enthalten.

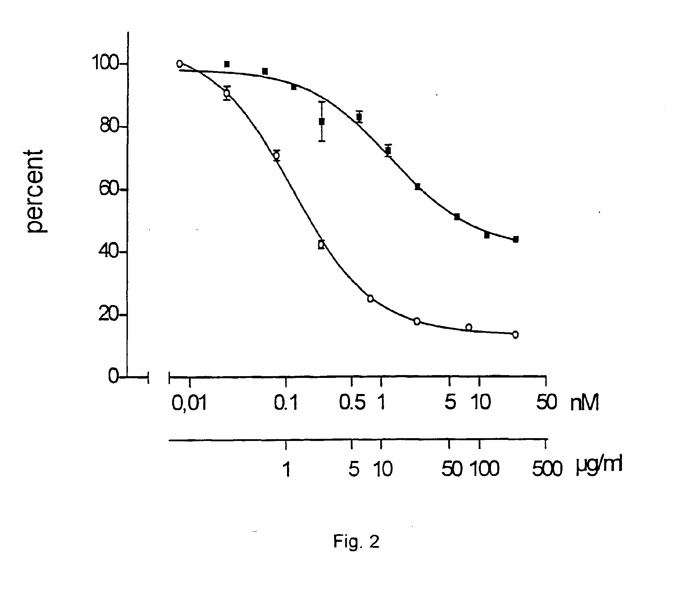

Um diesen Befund noch stärker zu untermauern, wurde der gleiche Assay mit einer Rezeptor-Präparation aus humanem Endometrium durchgeführt. Wiederum liegen beide ER-Subtypen in der Lösung vor. Die Ergebnisse dieser Untersuchungen sind in Fig. 2 gezeigt.

Figur 2 zeigt die Verdrängungskurven von radioaktiv markiertem Estradiol von ER aus humanem Endometrium durch Estradiol (E₂, offene Symbole) und VAC-Extrakte (dunkle Symbole) . Die Ordinate gibt den prozentualen Anteil der (noch verbleibenden) Bindung von radioaktiv markiertem Estradiol an den Estrogenrezeptor an. Die obere Abszisse bezeichnet die E₂-Konzentration, während die untere Abszisse die VAC-Konzentration wiedergibt. Diejenige Verdrängung, welche mit der niedrigsten Konzentration der Testsubstanzen erhalten wurde, wird als 100% definiert. Mittelwerte ± SEM wurden aus einer Dreifachbestimmung erhalten. Die EC₅₀ von VAC beträgt 12,7 µg/ml.

Die Ergebnisse zeigen klar, daß Phytoestrogene aus VAC-Extrakten an die humanen Estrogenrezeptoren binden, was das molekulare Initialereignis bei einer estrogenartigen Wirkung von Verbindungen aus VAC beim Menschen ist.

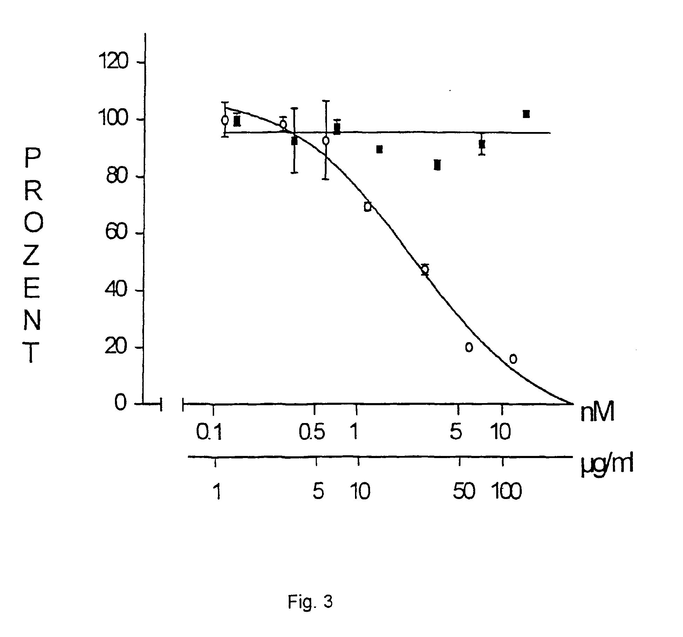

Aufgrund der Verfügbarkeit von rekombinanten Rezeptorproteinen können, subtypspezifische ER Assays (ER-alpha oder ER-beta) durchgeführt werden. Das Ergebnis in Form einer typischen ER-alpha-Verdrängungskurve ist in Fig. 3 gezeigt.

Die Figur 3 zeigt Dosis/Wirkungskurven von Estradiol (E₂, offene Symbole) und den VAC Extrakten der Anmelderin (dunkle Symbole) in einem Assay mit rekombinantem humanen ER-alpha. Die obere Abszisse gibt die E₂ - Konzentration an, während die untere die VAC - Konzentration angibt. Diejenige Bindung, welche mit der niedrigsten Konzentration der Testsubstanzen erhalten wurde, wurde mit 100% definiert. Mittelwerte ± SEM wurden aus Dreifachbestimmungen pro Konzentrationswert erhalten.

Während E₂ eine dosisbezogene Konkurrenz zu dem radioaktiven Rezeptorliganden zeigt, binden die Phytoestrogene des VAC-Extraktes nicht an den ER-alpha. Somit konnte keine EC₅₀ berechnet werden.

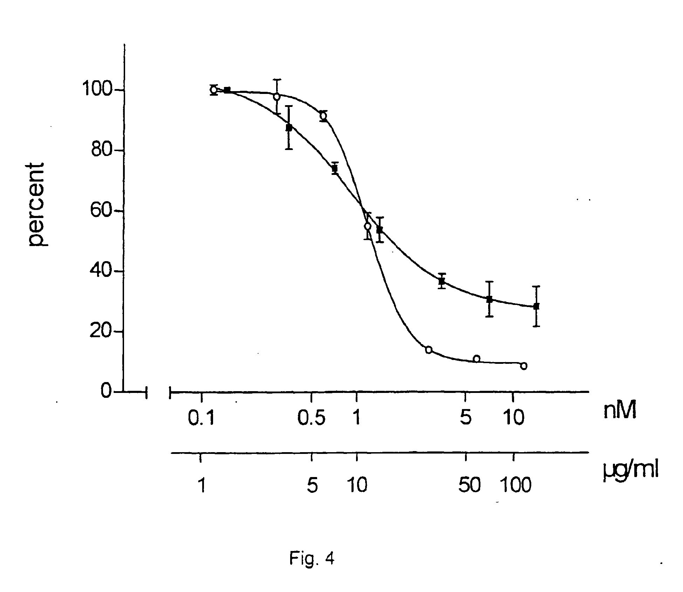

Mit denselben Testverbindungen wie beim ER-alpha-Assay, wurden nun ER-beta-Assays durchgeführt. Die Daten und der Graph eines repräsentativen Experimentes sind in Fig. 4 gezeigt.

Die Figur 4 zeigt Dosis/Wirkungskurven von Estradiol (E₂, offene Symbole) und den VAC Extrakten der Anmelderin (dunkle Symbole) in einem Assay mit rekombinantem humanen ER-beta. Die obere Abszisse gibt die E₂ - Konzentration an, während die untere die VAC - Konzentration angibt. Diejenige Verdrängung, welche mit der niedrigsten Konzentration der Testverbindungen erhalten wurde, wurde als 100% definiert. Mittelwerte ± SEM wurden aus Dreifachbestimmungen pro Konzentrationswert erhalten. Die EC₅₀ von VAC beträgt 9,9 µg/ml.

Im Gegensatz zum ER-alpha-Assay binden die Phytoestrogene der VAC-Extrakte der Anmelderin in dosisabhängiger Weise an den ER-beta. Diese Daten bestätigen ohne jeden Zweifel die ER-beta-Selektivität der Vitex Agnus castus Extrakte der Anmelderin.

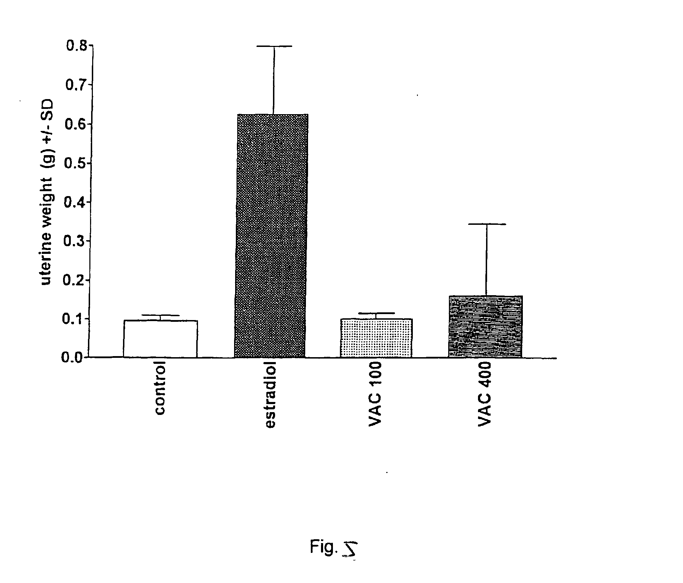

Zur Überprüfung des uterotropen Effektes wurde folgendes Experiment durchgeführt, dessen Daten graphisch in Fig. 5 gezeigt sind.

Über drei Monate wurden zwei unterschiedliche Konzentration von VAC-Extrakten (100mg/kg = VAC100 und 400mg/kg Körpergewicht = VAC400) dem Futter von ovariektomierten Rattenweibchen und orchidektomierten Männchen beigemischt. VAC hatte im Gegensatz zu Estradiol keinen uterotropen Einfluß und auch das Vaginalepithel blieb unbeeinflußt. Beide proliferativen Effekte des Estradiols sind im Rahmen der peri- und postklimatkterischen Estrogenersatztherapie bzw. einer Hormonersatztherapie bei Frauen nach einer Ovariektomie unerwünscht, da bei ständiger Gabe von Östrogenen und fehlender Gegenregulation durch Gestagene eine Hyperplasie des Endometriums durch den andauernden proliferativen Effekt des Estradiols nicht ausgeschlossen werden kann.

In Fig. 5 sind auf der Ordinate die Uterusgewichte der Ratten aufgetragen. Die einzelnen Balken betreffen die Kontrollgruppe (nur ovariektomiert), eine Estradiol-Positivkontrolle sowie die Effekte mit zwei unterschiedlichen VAC-Extraktkonzentrationen.

Eine uterotrope Wirkung wie beim Estradiol konnte gemäß Fig. 5 bei den mit VAC behandelten Tieren nicht festgestellt werden, das bedeutet, daß VAC nicht proliferativ auf das Uterusgewebe wirkt.

Jedoch wurde bei weiteren Untersuchungen überraschend gefunden, daß VAC, estrogene Effekte auf andere Organsysteme ausüben kann.

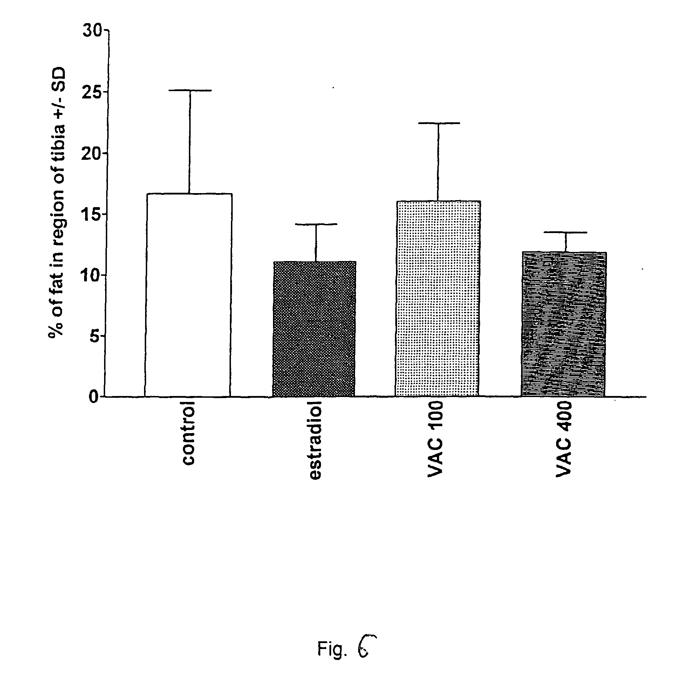

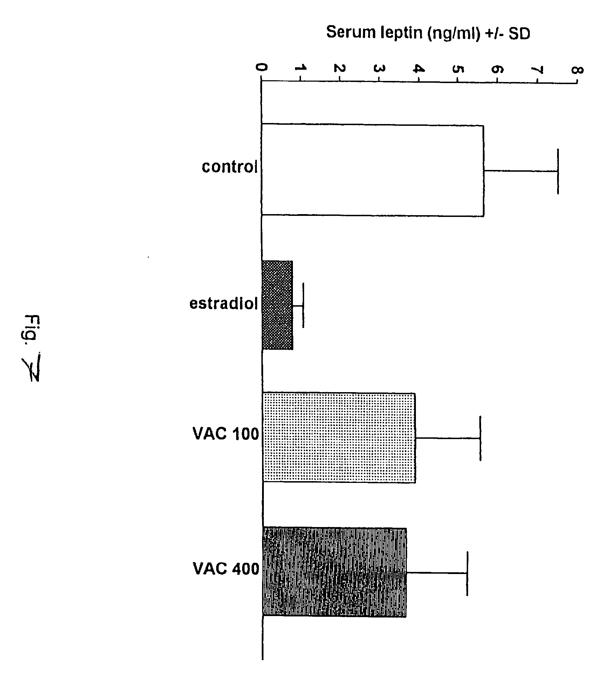

Mit computerassistierter Tomographie wurde das abdominale und paratibiale Fettgewebe bei männlichen und weiblichen Ratten bestimmt. Unter E2 und der hohen Dosis von Vitex Agnus castus ist eine Reduktion beider Gewebsanteile beobachtet worden. Gleichzeitig sind die Serum-Leptinspiegel der VAC behandelten Tiere abgesenkt. Die Ergebnisse dieser Untersuchung sind in den Figuren 6 und 7 gezeigt.

Fig. 6 zeigt die Wirkung von Estradiol und zwei unterschiedlichen VAC-Konzentrationen auf das paratibiale Fettgewebe, dargestellt als prozentualer Flächenanteil des Fettgewebes einer CT-Aufnahme im Bereich der Tibia gegenüber einer Kontrolle.

Fig. 7 zeigt die Wirkung von Estradiol und zwei unterschiedlichen VAC-Konzentrationen auf den Serumleptingehalt, auf der Ordinate aufgetragen in ng/ml.

Wie aus den Figuren 6 und 7 ersichtlich, zeigt sich eine Senkung des paratibialen Fettgehaltes und eine Senkung der Serumleptinkonzentration unter VAC-Gabe.

Ebenfalls mit computerassistierter Tomographie wurde die Veränderung der Knochendichte (geringere Osteoporoseentwicklung) an der Metaphyse der Tibia bei Rattenmännchen und -weibchen beobachtet. Es zeigte sich, daß unter VAC die Knochendichte deutlich weniger abnimmt, als bei den unbehandelten Kontrolltieren.

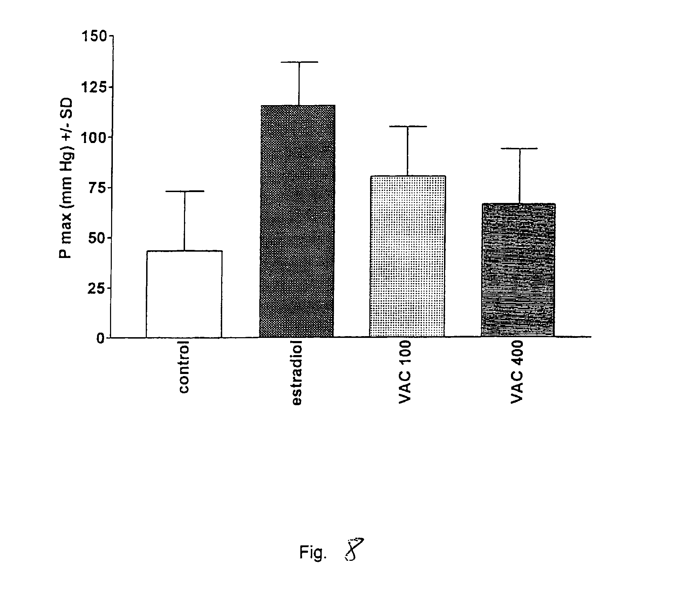

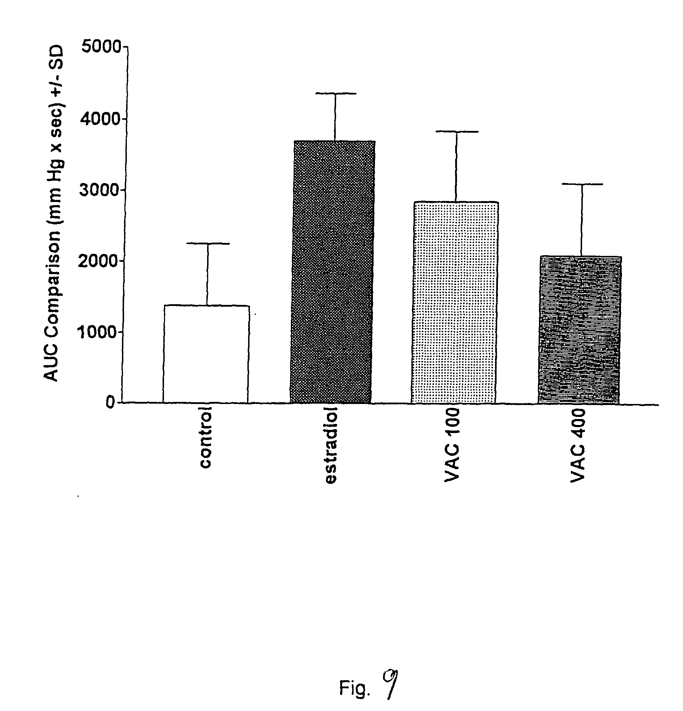

Die Instillation von 1 ml physiologischer Kochsalzlösung mit einem Katheter in die Blase weiblicher ovariektomierter Ratten innerhalb von 1 min führt zu einem Anstieg des Blaseninnendruckes. Bei Tieren die drei Monate lang mit E2-substituiert waren fällt der Druckanstieg etwa dreifach höher aus als bei den scheinbehandelten Kontrolltieren und ist geringfügig schwächer aber signifikant als bei den Estradiolbehandelten Tieren, wenn die Tiere mit VAC behandelt wurden. Die Ergebnisse dieser Untersuchung sind in den Figuren 8 und 9 gezeigt.

In Fig. 8 ist der maximale Blaseninnendruck in mmHg auf der Ordinate angegeben, der für eine Kontrolle, Estradiol als Positivkontrolle und zwei unterschiedliche VAC-Konzentrationen gemessen wurde.

Fig. 9 zeigt dagegen auf der Ordinate die Fläche unter der Druckmeßkurve [AUC] in mmHg x sec für eine Kontrolle, Estradiol als Positivkontrolle und zwei unterschiedliche VAC-Konzentrationen.

Nach alledem ist es somit möglich, VAC-Extrakte zur Herstellung von Arzneimitteln zur Behandlung von:
- Fettleibigkeit und dadurch einer möglichen Beeinflussung des metabolischen Syndroms, insbesondere Hypertonus, Arteriosklerose, Herzinfarkt, Hyperandrogenämie ohne uterotrope Wirkung einzusetzen.

### Literatur:

1.) Hrabovszky E, Shughrue PJ, Merchenthaler I, Hajszan T, Carpenter CD, Liposits Z, Petersen SL Detection of estrogen receptor-beta messenger ribonucleic acid and 125l-estrogen binding sites in luteinizing hormone-releasing hormone neurons of the rat brain. Endocrinology 2000 Sep; 141 (9):3506-9
2.) Hosokawa K, Ottander U, Wahlberg P, Ny T, Cajander S, Olofsson IJ. Dominant expression and distribution of oestrogen receptor beta over oestrogen receptor alpha in the human corpus luteum. Mol Hum, Reprod 2001 Feb;7(2):137-45
3.) Taylor AH, Al-Azzawi F. Immunolocalisation of oestrogen receptor beta in human tissues. J Mol Endocrinol 2000 Feb;24(1):145-55
4.) Kuiper GG, Carlsson B, Grandien K, Enmark E, Haggblad J, Nilsson S, Gustafsson JA 1997 Comparison of the ligand binding specificity and transcript tissue distribution of estrogen receptors alpha and beta. Endocrinology 138:863-870
5.) Saunders PT, Maguire SM, Gaughan J, Millar MR, 1997 Expression of oestrogen receptor beta (ER beta) in multiple rat tissues visualised by immunohistochemistry. J Endocrinol 154:R13-R16
6.) Mäkelä S, Strauss L, Kuiper G, Valve E, Salmi S, Santti R, Gustafsson JA, 2000 Differential expression of estrogen receptors alpha and beta in adult rat accessory sex glands and lower urinary tract. Mol Cell Endocrinol 164:109-116

## Patentansprüche

1. Verwendung von phytoestrogenhaltigen Extrakten ausschließlich aus Vitex agnus castus zur Herstellung eines Medikaments zur selektiven Modulation des Estrogen-Rezeptors-beta ohne uterotrope Wirkung, **dadurch gekennzeichnet, dass** die selektive Modulation des Estrogen-Rezeptors-beta zur Behandlung von Krankheitsbildern und pathophysiologischen Zuständen der
- Fettleibigkeit und dadurch mögliche Beeinflussung des metabolischen Syndroms, insbesondere Hypertonus, Arteriosklerose, Herzinfarkt, Hyperandrogenämie eingesetzt wird, wobei die Extrakte durch ethanolische Extraktion herstellbar sind.

2. Verwendung nach Anspruch 1, **dadurch gekennzeichnet, dass** der Extrakt in pharmazeutisch üblicher Form verwendet wird.

3. Verwendung nach Anspruch 3, **dadurch gekennzeichnet, dass** als pharmazeutische Formulierung Tropfen, Tabletten, Filmtabletten, Kapseln, Granulate, Dragees, Suppositorien, Salben, Cremes verwendet werden.

4. Verwendung nach einem der Ansprüche 1 bis 3, **dadurch gekennzeichnet, dass** die Extrakte aus Vitex agnus castus aus deren Früchten und/oder Blättern, herstellbar sind.

## Claims

1. Use of phytoestrogen-containing extracts exclusively from *Vitex agnus castus,* for preparing a medicament for selectively modulating the estrogen receptor beta without producing a uterotropic effect, **characterized in that** the selective modulation of the estrogen receptor beta is used in the treatment of clinical situations and pathophysiological conditions of obesity and an influence on the metabolic syndrome thereby possible, particularly hypertension, arteriosclerosis, cardiac infarct, hyperandrogenemia, said extracts being obtainable by ethanol extraction.

2. Use according to claim 1, **characterized in that** the extract is used in a standard pharmaceutical formulation.

3. Use according to claim 2, **characterized in that** drops, tablets, coated tablets, capsules, granulates, dragées, suppositories, ointments, creams are used as pharmaceutical formulations.

4. Use according to any one of claims 1 to 3, **characterized in that** the extracts from *Vitex agnus castus* are obtainable from the fruits and/or leaves thereof.

## Revendications

1. Utilisation d'extraits contenant des phyto oestrogènes constitués exclusivement de *Vitex agnus castus* pour la fabrication d'un médicament destiné à la modulation sélective du récepteur bêta des oestrogènes sans action utérotrope, **caractérisée en ce que** la modulation sélective du récepteur bêta des oestrogènes est mise en oeuvre pour le traitement des tableaux pathologiques et des états physiopathologiques de l'obésité et ainsi pour une influence possible sur le syndrome métabolique, notamment l'hypertonie, l'artériosclérose, l'infarctus du myocarde, l'hyperandrogénémie, où les extraits peuvent être fabriqués par extraction éthanolique.

2. Utilisation selon la revendication 1, **caractérisée en ce que** l'extrait est utilisé sous une forme pharmaceutiquement usuelle.

3. Utilisation selon la revendication 2, **caractérisée en ce que** l'on utilise comme formulation pharmaceutique les gouttes, les comprimés, les comprimés enrobés, les capsules, les granulés, les gélules, les suppositoires, les pommades, les crèmes.

4. Utilisation selon l'une des revendications 1 à 3, **caractérisée en ce que** les extraits de *Vitex agnus castus* peuvent être fabriqués à partir de ses fruits et/ou de ses feuilles.
